(19) Europäisches Patentamt
European Patent Office
Office européen des brevets

(11) EP 4 157 267 B1

(12) EUROPEAN PATENT SPECIFICATION

(45) Date of publication and mention of the grant of the patent:
**08.07.2026 Bulletin 2026/28**

(21) Application number: **21728553.5**

(22) Date of filing: **26.05.2021**

(51) International Patent Classification (IPC):
***A61K 31/4155*** *(2006.01)* ***A61K 31/506*** *(2006.01)*
***A61K 31/517*** *(2006.01)* ***A61K 31/519*** *(2006.01)*
***A61P 35/00*** *(2006.01)*

(52) Cooperative Patent Classification (CPC):
**A61K 31/506; A61K 31/4155; A61K 31/517; A61K 31/519; A61P 35/00**

(86) International application number:
**PCT/EP2021/063992**

(87) International publication number:
**WO 2021/239786 (02.12.2021 Gazette 2021/48)**

# (54) EGFR TKIS FOR USE IN THE TREATMENT OF NON-SMALL CELL LUNG CANCER

EGFR-TKIS ZUR VERWENDUNG BEI DER BEHANDLUNG VON NICHTKLEINZELLIGEM LUNGENKREBS

TKI DU EGFR DESTINÉS À ÊTRE UTILISÉS DANS LE TRAITEMENT DU CANCER DU POUMON NON À PETITES CELLULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **27.05.2020 US 202063030372 P**

(43) Date of publication of application:
**05.04.2023 Bulletin 2023/14**

(60) Divisional application:
**26172316.7**

(73) Proprietor: **Astrazeneca AB**
**151 85 Södertälje (SE)**

(72) Inventors:
- **RUKAZENKOV, Yuri**
  **Cambridge Cambridgeshire CB2 0AA (GB)**
- **GHIORGHIU, Serban**
  **Cambridge Cambridgeshire CB2 0AA (GB)**
- **BORELLINI, Flavia**
  **Cambridge Cambridgeshire CB2 0AA (GB)**
- **MANN, Helen**
  **Cambridge Cambridgeshire CB2 0AA (GB)**

(74) Representative: **AstraZeneca Intellectual Property**
**Eastbrook House**
**Shaftesbury Road**
**Cambridge CB2 8BF (GB)**

(56) References cited:
- **SULLIVAN I ET AL: "Osimertinib in the treatment of patients with epidermal growth factor receptor T790M mutation-positive metastatic non-small cell lung cancer: Clinical trial evidence and experience", THERAPEUTIC ADVANCES IN RESPIRATORY DISEASE, SAGE PUBLICATIONS LTD, UK, vol. 10, no. 6, 1 December 2016 (2016-12-01), pages 549 - 565, XP002786876, ISSN: 1753-4658**
- **CHENG HUA ET AL: "A meta-analysis of adjuvant EGFR-TKIs for patients with resected non-small cell lung cancer", LUNG CANCER, ELSEVIER, AMSTERDAM, NL, vol. 137, 5 August 2019 (2019-08-05), pages 7 - 13, XP085887894, ISSN: 0169-5002, [retrieved on 20190805], DOI: 10.1016/J.LUNGCAN.2019.08.002**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 157 267 B1

## Description

## FIELD

This specification describes epidermal growth factor receptor (EGFR) tyrosine kinase inhibitors (TKIs) for use in the adjuvant treatment after tumour resection of patients with epidermal growth factor receptor-mutation-positive (EGFRm) non-small cell lung cancer (NSCLC). In particular, the specification describes the use of either a second-generation or a third-generation EGFR TKI as an adjuvant therapy in this setting.

## BACKGROUND

Primary lung cancer is the most common form of cancer worldwide (approximately 13.5% of all new cancers cases in 2018) and it remains the leading cause of cancer-related death globally (25.3% of all deaths from cancer). Non-small cell lung cancer (NSCLC) represents approximately 80% to 90% of all lung cancers (National Comprehensive Cancer Network (NCCN) guidelines 2019 for NSCLC). Approximately 30% of all patients with NSCLC present with early stage (I-IIIA) disease, with surgery as the primary treatment. Although patients treated with surgery have a better prognosis, the 5-year survival for patients treated with surgery alone is low, ranging from 57% (stage IB) to 23% (stage IIIA). In an attempt to improve survival rates, adjuvant post-operative platinum-based chemotherapy has become a standard of care in patients with resected stage II and III NSCLC and select patients with resected stage IB disease. Pignon et al. (J Clin Oncol 2008;26:3552-9) found a 5-year absolute benefit of 5.4% from surgery and adjuvant chemotherapy, but recurrence or death rates in these patients remain high with 5-year overall survival (OS) rates ranging from 60-74% (stage I) to 38% (stage IIIA). More effective treatments are therefore needed.

In 2004, it was reported that activating mutations in exons 18-21 of EGFR correlated with a response to EGFR-TKI therapy in NSCLC (Science [2004], vol. 304, 1497-1500; New England Journal of Medicine [2004], vol. 350, 2129-2139). It is estimated that these mutations are prevalent in approximately 10-16% of NSCLC human patients in the United States and Europe, and in approximately 30-50% of NSCLC human patients in Asia. Two of the most significant EGFR activating mutations are exon 19 deletions and missense mutations in exon 21. Exon 19 deletions account for approximately 45% of known EGFR mutations. Eleven different mutations, resulting in deletion of three to seven amino acids, have been detected in exon 19, all centred around the uniformly deleted codons for amino acids 747-749. The most significant exon 19 deletion is E746-A750. The missense mutations in exon 21 account for approximately 39-45% of known EGFR mutations, of which the substitution mutation L858R accounts for approximately 39% of the total mutations in exon 21 (J. Thorac. Oncol. [2010], 1551-1558).

Two first generation (erlotinib & gefitinib), two second generation (afatinib & dacomitinib) and a third generation (osimertinib) epidermal growth factor receptor (EGFR) tyrosine kinase inhibitors (TKIs) are currently available for the management of advanced (metastatic) EGFR mutation-positive NSCLC. All these TKIs are effective in patients with NSCLC whose tumours harbour the in-frame deletions in exon 19 and the L858R point mutation in exon 21. These two mutations represent approximatively 90% of all EGFR mutations. In approximately 50% of patients, resistance to first- and second-generation EGFR TKI is mediated by the acquisition of the 'gatekeeper' mutation T790M. Currently, osimertinib is the only registered EGFR TKI that is active against exon 19 deletions and L858R mutation, regardless of the presence of T790M mutation.

Although studies of first generation EGFR TKIs in the adjuvant setting have been carried out (Cheng et al. Lung Cancer 2019;137:7-13; Zhong et al. Lancet Oncol 2018), the role of EGFR TKIs in adjuvant therapy following complete surgical resection of the tumour remains under investigation. The utility of second and third generation EGFR TKIs in this setting remains to be explored.

Sullivan et al. (Therapeutic Advances in Respiratory Disease, 2016, vol. 10, no. 6, pages 549-565) describes osimertinib in the treatment of patients with epidermal growth factor receptor T790M mutation-positive metastatic non-small cell lung cancer, referencing an ongoing ADAURA clinical trial.

Cheng et al. (Lung Cancer, 2019, vol. 137, pages 7-13) describes a meta-analysis to compare adjuvant EGFR-TKIs with a placebo or adjuvant chemotherapy among patients with resected non-small cell lung cancer.

Osimertinib is a third-generation EGFR TKI with superior efficacy to first-generation EGFR TKIs in EGFRm advanced NSCLC. The Phase III FLAURA study (N. Engl. J Med. 2018, 378, 113-25; N Engl J Med. 2020, 382(1):41-50) comparing the efficacy and safety of osimertinib administered as first-line therapy to patients with advanced mutation-positive EGFR (Ex19del or L858R) NSCLC versus (vs.) either gefitinib or erlotinib showed a significantly improved median progression free survival (PFS) in the osimertinib arm (18.9 months [95% confidence interval [Cl]: 15.2, 21.4]) compared to erlotinib or gefitinib (10.2 months [95% CI: 9.6, 11.1]), with a hazard ratio (HR) of 0.46 (95% CI: 0.37, 0.57; $p < 0.0001$). The median overall survival was 38.6 months (95% confidence interval [Cl], 34.5 to 41.8) in the osimertinib group and 31.8 months (95% CI, 26.6 to 36.0) in the comparator group (hazard ratio for death, 0.80; 95.05% CI, 0.64 to 1.00; $P = 0.046$). On the basis on the results of the FLAURA study, osimertinib is recommended by the NCCN Panel as preferred first-line

therapy in these patients. Of note, in the FLAURA study, irrespective of status with respect to known or treated Central Nervous System (CNS) metastases at trial entry, events of CNS progression were observed in 6% patients in the osimertinib group and 15% in the standard EGFR TKI group. Moreover, in patients with CNS metastases on a baseline brain scan, osimertinib demonstrated a nominally statistically significant and clinically meaningful improvement in CNS PFS over standard EGFR-TKIs with a 52% reduction in the risk of CNS progression (HR 0.48;95%CI 0.26-0.86 p=0.014; median CNS PFS not reached (95% CI 16.5,NC (i.e. could Not be Calculated)) vs 13.9 months (95% CI 8.3 to NC); J Clin Oncol. [2018], vol. 36(33), 3290-7).

ADAURA (NCT02511106; Clinical Lung Cancer [2018], Vol. 19, No. 4, e533-36) is a Phase 3, double-blind, randomised study assessing the efficacy and safety of osimertinib vs placebo in patients classified with stage IB-IIIA EGFRm NSCLC after complete surgical resection and adjuvant chemotherapy, when indicated. Unexpectedly, following an Independent Data Monitoring Committee recommendation, ADAURA was unblinded early due to overwhelming efficacy. We have found that osimertinib demonstrates a statistically significant and clinically meaningful improvement in disease free survival (DFS) in patients classified with stage IB/II/IIIA EGFRm NSCLC in the adjuvant setting.

## SUMMARY

In a first aspect, the present specification describes an EGFR TKI for use in the adjuvant treatment after tumour resection of a patient with epidermal growth factor receptor-mutation-positive (EGFRm) non-small cell lung cancer (NSCLC), wherein the EGFR TKI is either a second-generation or third-generation EGFR TKI.

In a further aspect, the present specification describes an EGFR TKI for use in the adjuvant treatment of a patient classified with stage IB, II or IIIA epidermal growth factor receptor-mutation-positive (EGFRm) non-small cell lung cancer (NSCLC) following complete tumour resection, wherein the EGFR TKI is either a second-generation or third-generation EGFR TKI.

In a further aspect, there is provided a second-generation or third-generation EGFR TKI for use in a method of treating a patient with epidermal growth factor receptor-mutation-positive (EGFRm) non-small cell lung cancer (NSCLC), said method comprising the adjuvant treatment after tumour resection of the patient with a second-generation or third-generation EGFR TKI.

In a further aspect, there is provided a second-generation or third-generation EGFR TKI for use in a method of treating a patient classified with stage IB, II or IIIA epidermal growth factor receptor-mutation-positive (EGFRm) non-small cell lung cancer (NSCLC) following complete tumour resection, said method comprising the adjuvant treatment of the patient with a second-generation or third-generation EGFR TKI.

In a further aspect, there is provided a second-generation or third-generation EGFR TKI for use in a method of improving disease-free survival (DFS) in a patient with epidermal growth factor receptor-mutation-positive (EGFRm) non-small cell lung cancer (NSCLC), the method comprising the adjuvant treatment after tumour resection of the patient with a second-generation or third-generation EGFR TKI.

In a further aspect, there is provided a second-generation or third-generation EGFR TKI for use in a method of improving disease-free survival (DFS) in a patient classified with stage IB, II or IIIA epidermal growth factor receptor-mutation-positive (EGFRm) non-small cell lung cancer (NSCLC) following complete tumour resection, the method comprising the adjuvant treatment of the patient with a second-generation or third-generation EGFR TKI.

The specification further describes such treatment wherein the adjuvant EGFR TKI treatment results in one or more of improved disease free survival (DFS), delayed time to first subsequent therapy (TFST), or improved overall survival (OS).

## DESCRIPTION OF FIGURES

Figure 1: Kaplan-Meier (KM) plot of DFS in overall ADAURA patient population (stage IB/II/IIIA)
Figure 2: KM plot of DFS in ADAURA patients with stage II-IIIA disease
Figure 3: KM plot of DFS in ADAURA patients with stage IB disease
Figure 4: KM plot of DFS in ADAURA patients with stage II disease
Figure 5: KM plot of DFS in ADAURA patients with stage IIIA disease
Figure 6: DFS across subgroups in the overall ADAURA population
Figure 7: KM plot of DFS in ADAURA patients who had received prior adjuvant chemotherapy
Figure 8: KM plot of DFS in ADAURA patients who had not received prior adjuvant chemotherapy

## DETAILED DESCRIPTION

As used herein, the term "about" when referring to any given numerical value means within ±10%, ±5%, or ±2% of that value.

### EGFR mutation positive NSCLC and diagnostic methods

The skilled person will be aware of the mutations in EGFR which correlate with an improved response to EGFR-TKI therapy. In aspects of this specification, the EGFR mutation-positive NSCLC comprises activating mutations in EGFR, either alone or in combination with other EGFR mutations including T790M. In further aspects, the activating mutations in EGFR comprise activating mutations in exons 18-21, either alone or in combination with other EGFR mutations including T790M. In further aspects, the activating mutations in EGFR comprise exon 19 deletions or missense mutations in exon 21, either alone or in combination with other EGFR mutations including T790M. In further aspects, the activating mutations in EGFR comprise exon 19 deletions or exon 21 L858R substitution mutations, either alone or in combination with other EGFR mutations including T790M. In further aspects, the activating mutations in EGFR comprise exon 19 deletions or L858R substitution mutations. In further aspects, the activating mutations in EGFR comprise exon 19 deletions or exon 21 L858R substitution mutations.

The skilled person will be aware of numerous methods to detect EGFR activating mutations. Tests suitable for use in these methods have been approved by the US Food and Drug Administration (FDA). These include both tumour tissue and plasma based diagnostic methods. In general, the EGFR mutation status is assessed using a tumour tissue sample derived from the patient. A particular example of a suitable diagnostic test to detect EGFR activating mutations, and in particular to detect exon 19 deletions or exon 21 L858R substitution mutations, is the Cobas™ EGFR Mutation Test v2 (Roche Molecular Diagnostics).

In aspects, therefore, the EGFR mutation-positive NSCLC comprises activating mutations in EGFR (such as activating mutations in exons 18-21, for example exon 19 deletions or missense mutations in exon 21, for example exon 19 deletions or exon 21 L858R substitution mutations), wherein the EGFR mutation status of the patient has been determined using an appropriate diagnostic test. In further aspects, the EGFR mutation status has been determined using a tumour tissue sample. In further aspects, the EGFR mutation status has been determined using a plasma sample. In further aspects, the diagnostic method uses an FDA-approved test. In further aspects, the diagnostic method uses the Cobas™ EGFR Mutation Test (v1 or v2).

### EGFR TKIs

EGFR TKIs can be characterised as either first-, second- or third-generation EGFR TKIs, as set out below.

First-generation EGFR TKIs are reversible inhibitors of EGFR bearing activating mutations that do not significantly inhibit EGFR bearing the T790M mutation. Examples of first-generation TKIs include gefitinib and erlotinib.

Second-generation EGFR TKIs are irreversible inhibitors of EGFR bearing activating mutations that do not significantly inhibit EGFR bearing the T790M mutation. Examples of second-generation TKIs include afatinib and dacomitinib.

Third-generation EGFR TKIs are inhibitors of EGFR bearing activating mutations that also significantly inhibit EGFR bearing the T790M mutation and do not significantly inhibit wild-type EGFR. Examples of third-generation TKIs include compounds of Formula (I), osimertinib, AZD3759, lazertinib, nazartinib, CO1686 (rociletinib), HM61713, ASP8273, EGF816, PF-06747775 (mavelertinib), avitinib (abivertinib), alflutinib (AST2818) and CK-101 (RX-518), HS-10296 and BPI-7711. Further examples include oritinib (SH-1028), befortinib (D-0316), ASK-120067, ZN-e4, YZJ-0318, TL007 XZP (kenaitinib), YK-029A, SLC005-I, TY-9591, XZP-5809-TT1, ZSP0391, and TQB3456.

In an aspect, the EGFR TKI is a second-generation EGFR TKI. In a further aspect, the second-generation EGFR TKI is selected from dacomitinib, or a pharmaceutically acceptable salt thereof, and afatinib or a pharmaceutically acceptable salt thereof.

In an aspect, the EGFR TKI is a third-generation EGFR TKI. In a further aspect, the third-generation EGFR TKI is a compound of Formula (I), as defined below. In a further aspect, the third-generation EGFR TKI is selected from the group consisting of osimertinib or a pharmaceutically acceptable salt thereof, AZD3759 or a pharmaceutically acceptable salt thereof, lazertinib or a pharmaceutically acceptable salt thereof, abivertinib or a pharmaceutically acceptable salt thereof, alflutinib or a pharmaceutically acceptable salt thereof, CK-101 or a pharmaceutically acceptable salt thereof, HS-10296 or a pharmaceutically acceptable salt thereof and BPI-7711 or a pharmaceutically acceptable salt thereof. In a further aspect, the third generation EGFR TKI is osimertinib or a pharmaceutically acceptable salt thereof.

Compounds of Formula (I)

In an aspect, the EGFR TKI is a compound of Formula **(I):**

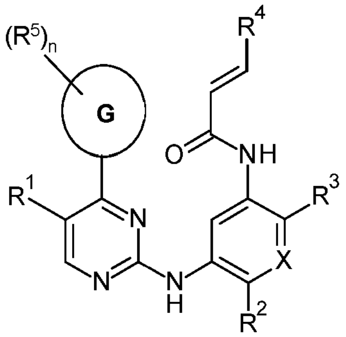

**(I)**

wherein:

**G** is selected from 4,5,6,7-tetrahydropyrazolo[1,5-*a*]pyridin-3-yl, indol-3-yl, indazol-1-yl, 3,4-dihydro-1H-[1,4]oxazino[4,3-a]indol-10-yl, 6,7,8,9-tetrahydropyrido[1,2-a]indol-10-yl, 5,6-dihydro-4H-pyrrolo[3,2,1-ij]quinolin-1-yl, pyrrolo[3,2-b]pyridin-3-yl and pyrazolo[1,5-*a*]pyridin-3-yl;

$\mathbf{R^1}$ is selected from hydrogen, fluoro, chloro, methyl and cyano;

$\mathbf{R^2}$ is selected from methoxy, trifluoromethoxy, ethoxy, 2,2,2-trifluoroethoxy and methyl;

$\mathbf{R^3}$ is selected from (3*R*)-3-(dimethylamino)pyrrolidin-1-yl, (3*S*)-3-(dimethyl-amino)pyrrolidin-1-yl, 3-(dimethylamino)azetidin-1-yl, [2-(dimethylamino)ethyl]-(methyl)amino, [2-(methylamino)ethyl](methyl)amino, 2-(dimethylamino)ethoxy, 2-(methylamino)ethoxy, 5-methyl-2,5-diazaspiro[3.4]oct-2-yl, (3a*R*,6a*R*)-5-methylhexa-hydro-pyrrolo[3,4-*b*]pyrrol-1(2*H*)-yl, 1-methyl-1,2,3,6-tetrahydropyridin-4-yl, 4-methylpiperizin-1-yl, 4-[2-(dimethylamino)-2-oxoethyl]piperazin-1-yl, methyl[2-(4-methylpiperazin-1-yl)ethyl]amino, methyl[2-(morpholin-4-yl)ethyl]amino, 1-amino-1,2,3,6-tetrahydropyridin-4-yl and 4-[(2*S*)-2-aminopropanoyl]piperazin-1-yl;

$\mathbf{R^4}$ is selected from hydrogen, 1-piperidinomethyl and N,N-dimethylaminomethyl;

$\mathbf{R^5}$ is independently selected from methyl, ethyl, propyl, 2,2-difluoroethyl, 2,2,2-trifluoroethyl, fluoro, chloro and cyclopropyl;

**X** is CH or N; and

**n** is 0, 1 or 2;

or a pharmaceutically acceptable salt thereof.

In a further aspect there is provided a compound of Formula **(I)**, as defined above, wherein **G** is selected from indol-3-yl and indazol-1-yl; $\mathbf{R^1}$ is selected from hydrogen, fluoro, chloro, methyl and cyano; $\mathbf{R^2}$ is selected from methoxy and 2,2,2-trifluoroethoxy; $\mathbf{R^3}$ is selected from[2-(dimethylamino)ethyl]-(methyl)amino, [2-(methylamino)ethyl](methyl)amino, 2-(dimethylamino)ethoxy and 2-(methylamino)ethoxy; $\mathbf{R^4}$ is hydrogen; $\mathbf{R^5}$ is selected from methyl, 2,2,2-trifluoroethyl and cyclopropyl; **X** is CH or N; and **n is** 0 or 1; or a pharmaceutically acceptable salt thereof.

Examples of compounds of Formula (I) include those described in WO 2013/014448, WO 2015/175632, WO 2016/054987, WO 2016/015453, WO 2016/094821, WO 2016/070816 and WO 2016/173438.

Osimertinib and pharmaceutical compositions thereof

Osimertinib has the following chemical structure:

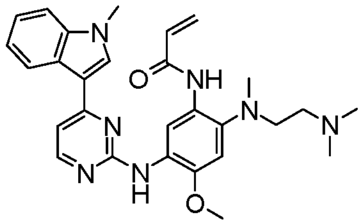

The free base of osimertinib is known by the chemical name: *N*-(2-{2-dimethylamino ethyl-methylamino}-4-methoxy-5-{[4-(1-methylindol-3-yl)pyrimidin-2-yl]amino}phenyl) prop-2-enamide. Osimertinib is described in WO 2013/014448. Osimertinib is also known as AZD9291.

Osimertinib may be found in the form of the mesylate salt: *N*-(2-{2-dimethylamino ethyl-methylamino}-4-methoxy-5-{[4-(1-methylindol-3-yl)pyrimidin-2-yl]amino}phenyl) prop-2-enamide mesylate salt. Osimertinib mesylate is also known as TAGRISSO™.

Osimertinib mesylate is currently approved as an oral once daily tablet formulation, at a dose of 80 mg (expressed as free base, equivalent to 95.4 mg osimertinib mesylate), for the treatment of metastatic EGFR T790M mutation positive NSCLC patients. A 40 mg oral once daily tablet formulation (expressed as free base, equivalent to 47.7 mg osimertinib mesylate) is available should dose modification be required.

The tablet core comprises pharmaceutical diluents (such as mannitol and microcrystalline cellulose), disintegrants (such as low-substituted hydroxypropyl cellulose) and lubricants (such as sodium stearyl fumarate). The tablet formulation is described in WO 2015/101791.

In an aspect, therefore, osimertinib, or a pharmaceutically acceptable salt thereof, is in the form of the mesylate salt, i.e. *N*-(2-{2-dimethylamino ethyl-methylamino}-4-methoxy-5-{[4-(1-methylindol-3-yl)pyrimidin-2-yl]amino}phenyl) prop-2-enamide mesylate salt.

In an aspect, osimertinib, or a pharmaceutically acceptable salt thereof, is administered once-daily. In a further aspect, osimertinib mesylate is administered once-daily.

In an aspect, the total daily dose of osimertinib is about 80 mg. In a further aspect, the total daily dose of osimertinib mesylate is about 95.4 mg.

In an aspect, the total daily dose of osimertinib is about 40 mg. In a further aspect, the total daily dose of osimertinib mesylate is about 47.7 mg.

In an aspect, osimertinib, or a pharmaceutically acceptable salt thereof, is in tablet form.

In an aspect, osimertinib, or a pharmaceutically acceptable salt thereof, is administered in the form of a pharmaceutical composition comprising one or more pharmaceutically acceptable excipients. In a further aspect, the composition comprises one or more pharmaceutical diluents (such as mannitol and microcrystalline cellulose), one or more pharmaceutical disintegrants (such as low-substituted hydroxypropyl cellulose) or one or more pharmaceutical lubricants (such as sodium stearyl fumarate).

In an aspect, the composition is in the form of a tablet, wherein the tablet core comprises: (a) from 2 to 70 parts of osimertinib, or a pharmaceutically acceptable salt thereof; (b) from 5 to 96 parts of two or more pharmaceutical diluents; (c) from 2 to 15 parts of one or more pharmaceutical disintegrants; and (d) from 0.5 to 3 parts of one or more pharmaceutical lubricants; and wherein all parts are by weight and the sum of the parts (a)+(b)+(c)+(d)=100.

In an aspect, the composition is in the form of a tablet, wherein the tablet core comprises: (a) from 7 to 25 parts of osimertinib, or a pharmaceutically acceptable salt thereof; (b) from 55 to 85 parts of two or more pharmaceutical diluents, wherein the pharmaceutical diluents comprise microcrystalline cellulose and mannitol; (c) from 2 to 8 parts of pharmaceutical disintegrant, wherein the pharmaceutical disintegrant comprises low-substituted hydroxypropyl cellulose; (d) from 1.5 to 2.5 parts of pharmaceutical lubricant, wherein the pharmaceutical lubricant comprises sodium stearyl fumarate; and wherein all parts are by weight and the sum of the parts (a)+(b)+(c)+(d)=100.

In an aspect, the composition is in the form of a tablet, wherein the tablet core comprises: (a) about 19 parts of osimertinib mesylate; (b) about 59 parts of mannitol; (c) about 15 parts of microcrystalline cellulose; (d) about 5 parts of low-substituted hydroxypropyl cellulose; and (e) about 2 parts of sodium stearyl fumarate; and wherein all parts are by weight and the sum of the parts (a)+(b)+(c)+(d)+(e)=100.

AZD3759

AZD3759 has the following chemical structure:

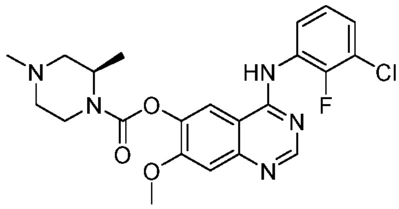

The free base of AZD3759 is known by the chemical name: 4-[(3-chloro-2-fluorophenyl)amino]-7-methoxy-6-quinazolinyl (2*R*)-2,4-dimethyl-1-piperazinecarboxylate. AZD3759 is described in WO 2014/135876.
In an aspect, AZD3759, or a pharmaceutically acceptable salt thereof, is administered twice-daily. In a further aspect, AZD3759 is administered twice-daily.
In an aspect, the total daily dose of AZD3759 is about 400 mg. In a further aspect, about 200 mg of AZD3759 is administered twice a day.

Lazertinib

Lazertinib has the following chemical structure:

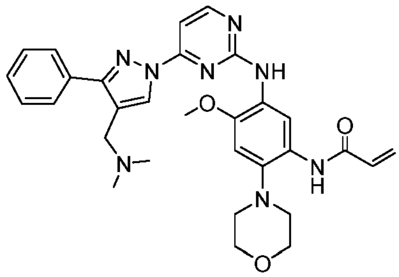

The free base of lazertinib is known by the chemical name *N*-{5-[(4-{4-[(dimethylamino)methyl]-3-phenyl-1H-pyrazol-1-yl}-2-pyrimidinyl)amino]-4-methoxy-2-(4-morpholinyl)phenyl}acrylamide. Lazertinib is described in WO 2016/060443. Lazertinib is also known by the names YH25448 and GNS-1480.
In an aspect, lazertinib, or a pharmaceutically acceptable salt thereof, is administered once-daily. In a further aspect, lazertinib is administered once-daily.
In an aspect, the total daily dose of lazertinib is about 20 to 320 mg.
In an aspect, the total daily dose of lazertinib is about 240 mg.

Avitinib (abivertinib)

Avitinib has the following chemical structure:

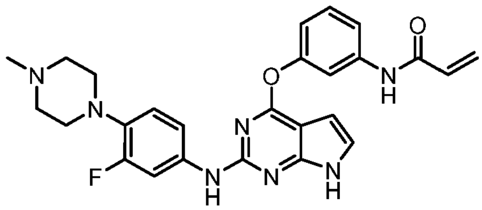

The free base of avitinib is known by the chemical name: N-(3-((2-((3-fluoro-4-(4-methylpiperazin-1-yl)phenyl) amino)-7H-pyrrolo(2,3-d)pyrimidin-4-yl)oxy)phenyl)prop-2-enamide. Avitinib is disclosed in US2014038940. Avitinib is also known as abivertinib.
In an aspect, avitinib or a pharmaceutically acceptable salt thereof, is administered twice daily. In a further aspect, avitinib maleate is administered twice daily.
In an aspect, the total daily dose of avitinib maleate is about 600 mg.

Alflutinib (furmonertinib)

Alflutinib has the following chemical structure:

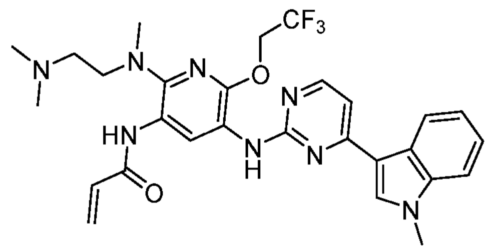

The free base of alflutinib is known by the chemical name: N-{2-{[2-(dimethylamino)ethyl](methyl)amino}-6-(2,2,2-trifluoroethoxyl)-5-{[4-(1-methyl-1H -indol-3-yl)pyrimidin-2-yl]amino}pyridin-3-yl}acrylamide. Alflutinib is disclosed in WO 2016/15453. Alflutinib is also known as AST2818.

In an aspect, alflutinib or a pharmaceutically acceptable salt thereof, is administered once daily. In a further aspect, alflutinib mesylate is administered once daily.

In an aspect, the total daily dose of alflutinib mesylate is about 80 mg.

In an aspect, the total daily dose of alflutinib mesylate is about 40 mg.

Afatinib

Afatinib has the following chemical structure:

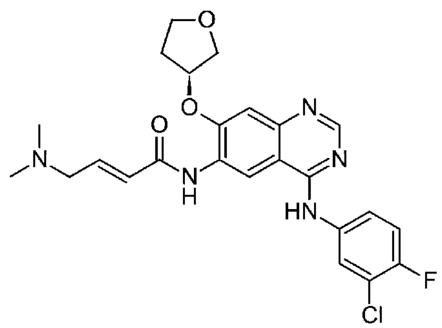

The free base of afatinib is known by the chemical name: *N*-[4-(3-chloro-4-fluoroanilino)-7-[(3S)-oxolan-3-yl]oxyquinazolin-6-yl]-4-(dimethylamino)but-2-enamide. Afatinib is disclosed in WO 02/50043. Afatinib is also known as Gilotrif.

In an aspect, afatinib or a pharmaceutically acceptable salt thereof, is administered once daily. In a further aspect, afatinib dimaleate is administered once daily.

In an aspect, the total daily dose of afatinib dimaleate is about 40 mg.

In an aspect, the total daily dose of afatinib dimaleate is about 30 mg.

CK-101

CK-101 has the following chemical structure:

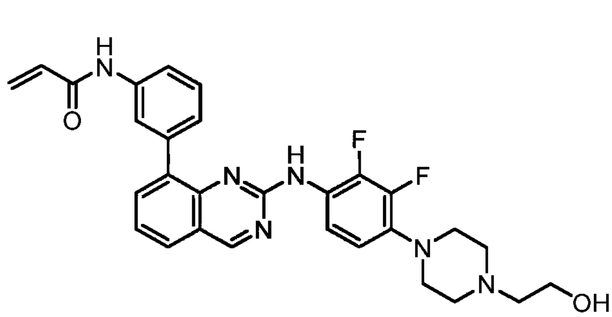

The free base of CK-101 is known by the chemical name: N-(3-(2-((2,3-difluoro-4-(4-(2-hydroxyethyl)piperazin-1-yl)phenyl)amino)quinazolin-8-yl)phenyl)acrylamide. CK-101 is disclosed in WO 2015/027222. CK-101 is also known as RX-518.

HS-10296 (almonertinib)

HS-10296 (almonertinib) has the following chemical structure:

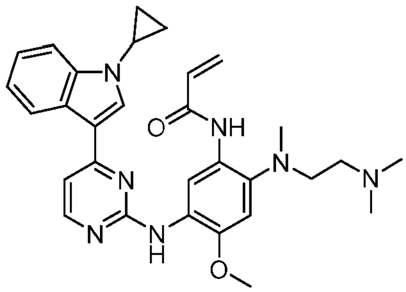

The free base of HS-10296 is known by the chemical name: N-[5-[[4-(1-cyclopropylindol-3-yl)pyrimidin-2-yl] amino]-2-[2-(dimethylamino)ethyl-methyl-amino]-4-methoxy-phenyl]prop-2-enamide. HS-10296 is disclosed in WO 2016/054987.

In an aspect, the total daily dose of HS-10296 is about 110 mg.

BPI-7711

BPI-7711 has the following chemical structure:

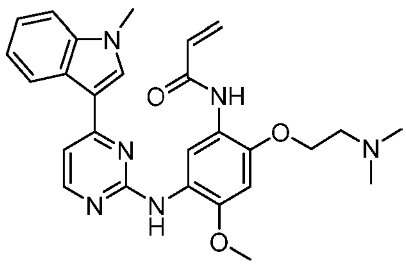

The free base of BPI-7711 is known by the chemical name: N-[2-[2-(dimethylamino)ethoxy]-4-methoxy-5-[[4-(1-methylindol-3-yl)pyrimidin-2-yl]amino]phenyl]prop-2-enamide. BPI-7711 is disclosed in WO 2016/94821.

In an aspect, the total daily dose of BPI-7711 is about 180 mg.

Dacomitinib

Dacomitinib has the following chemical structure:

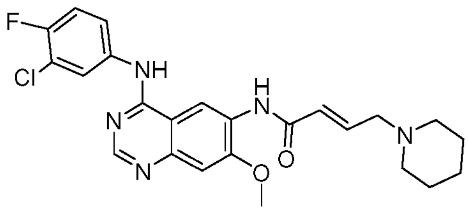

The free form of dacomitinib is known by the chemical name: (2*E*)-*N*-{4-[(3-chloro-4-fluorophenyl)amino]-7-methoxyquinazolin-6-yl}-4-(piperidin-1-yl)but-2-enamide. Dacomitinib is described in WO 2005/107758. Dacomitinib is also known by the name PF-00299804.

Dacomitinib may be found in the form of dacomitinib monohydrate, i.e. (2E)-N-{4-[(3-chloro-4-fluorophenyl) amino]-7-methoxyquinazolin-6-yl}-4-(piperidin-1-yl)but-2-enamide monohydrate.

In an aspect, dacomitinib, or a pharmaceutically acceptable salt thereof, is administered once-daily. In a further aspect, dacomitinib monohydrate is administered once-daily.

In an aspect, the total daily dose of dacomitinib monohydrate is about 45 mg.

In an aspect, dacomitinib, or a pharmaceutically acceptable salt thereof, is in tablet form.

In an aspect, dacomitinib, or a pharmaceutically acceptable salt thereof, is administered in the form of a pharmaceutical composition comprising one or more pharmaceutically acceptable excipients. In a further aspect, the one or more pharmaceutically acceptable excipients comprise lactose monohydrate, microcrystalline cellulose, sodium starch glycolate and magnesium stearate.

Nazartinib

Nazartinib has the following chemical structure:

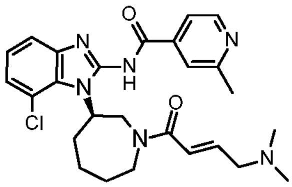

The free base of Nazartinib is known by the chemical name: N-(7-chloro-1-(1-(4-(dimethylamino)but-2-enoyl) azepan-3-yl)-1H- benzordlimidazol-2-yl)-2-methylisonicotinamide. Nazartinib is disclosed in WO 2013/184757.

In an aspect, the total daily dose of Nazartinib is about 150 mg, about 225 mg, or about 350 mg.

**Patients and Clinical Outcomes**

Patients with epidermal growth factor receptor-mutation-positive (EGFRm) non-small cell lung cancer (NSCLC), who may optionally be classified with classified with stage IB, II and IIIA disease, who receive an EGFR TKI after tumour resection for use according to the methods set out in this specification may benefit from an improved prognosis compared to existing standard of care, placebo, or no treatment. In particular, the adjuvant EGFR TKI treatment after tumour resection may provide one or more of improved disease free survival (DFS); delayed time to first subsequent therapy (TFST); or improved overall survival (OS).

DFS is defined as the time from randomisation (or initiation of treatment) until the date of disease recurrence, or death. Improvements in DFS may be measured relative to existing standard of care, placebo, or no treatment. In an aspect, DFS may be measured relative to placebo, or no treatment.

In an aspect, the adjuvant EGFR TKI treatment provides at least about a 50% (such as at least about 60%, at least about 70%, or at least about 80%) reduction in the risk of disease recurrence or death. In a further aspect, the patient may optionally have been classified with stage IB, II or IIIA EGFRm NSCLC following complete tumour resection and the adjuvant EGFR TKI treatment provides about a 75% (such as 76%, 77%, 78, 79%, 80% or 81%) reduction in the risk of disease recurrence or death. In a further aspect, the patient may optionally have been classified with stage IB, II or IIIA EGFRm NSCLC following complete tumour resection and the adjuvant EGFR TKI treatment provides about a 75% to about 90% (such as 77% to about 88%; or such as 77% to about 82%; or such as about 78% to about 80%) reduction in the risk of disease recurrence or death. In a further aspect, the patient may optionally have been classified with stage II or IIIA EGFRm NSCLC following complete tumour resection and the adjuvant EGFR TKI treatment provides about an 80% (such as 81%, 82%, 83%, 84% or 85%) reduction in the risk of disease recurrence or death. In a further aspect, the patient may optionally have been classified with stage II or IIIA EGFRm NSCLC following complete tumour resection and the adjuvant EGFR TKI treatment provides about an 80% to about 90% (such as about 80% to about 85%; or such as about 82% to about 84%) reduction in the risk of disease recurrence or death. In a further aspect, the patient may optionally have been classified with stage IB EGFRm NSCLC following complete tumour resection and the adjuvant EGFR TKI treatment provides about a 50% (such as 48%, 49%, 50%, 51% or 52%) reduction in the risk of disease recurrence or death. In a further aspect, the patient may optionally have been classified with stage IB EGFRm NSCLC following complete tumour resection and the adjuvant EGFR TKI treatment provides about an 45% to about 55% (such as about 48% to about 52%; or such as about 49% to about 51%) reduction in the risk of disease recurrence or death. In a further aspect, the patient may optionally have been classified with stage II EGFRm NSCLC following complete tumour resection and the adjuvant EGFR TKI treatment provides about an 80% (such as 81%, 82%, 83%, 84% or 85%) reduction in the risk of disease recurrence or death. In a further aspect, the patient may optionally have been classified with stage II EGFRm NSCLC following complete tumour resection and the adjuvant EGFR TKI treatment provides about an 80% to about 90% (such as about 80% to about 85%; or such as about 82% to about 84%) reduction in the risk of disease recurrence or death. In a further aspect, the patient may optionally have been classified with stage IIIA EGFRm NSCLC following complete tumour resection and the adjuvant EGFR TKI treatment provides about an 85% (such as 86%, 87%, 88%, 89% or 90%) reduction in the risk of disease recurrence or death. In a further aspect, the patient may optionally have been classified with stage IIIA EGFRm NSCLC following complete tumour resection and the adjuvant EGFR TKI treatment provides about an 85% to about 95%

(such as about 85% to about 90%; or such as about 87% to about 89%) reduction in the risk of disease recurrence or death.

In an aspect, the adjuvant EGFR TKI treatment provides DFS of at least about 12 months, such as at least about 24 months, such as at least about 36 months, such as at least about 48 months, or such as at least about 60 months.

In an aspect, the adjuvant EGFR TKI treatment provides a probability of DFS of about 97% (such as 95%, 96%, 97%, 98% or 99%) at about 12 months. In a further aspect, the adjuvant EGFR TKI treatment provides a probability of DFS of about 95% to about 99% (such as about 95% to about 98%; or such as about 96% to 98%) at about 12 months. In a further aspect, the adjuvant EGFR TKI treatment provides a probability of DFS of about 90% (such as 87%, 88%, 89%, 90%, 91% or 92%) at about 24 months. In a further aspect, the adjuvant EGFR TKI treatment provides a probability of DFS of about 85% to about 95% (such as about 86% to about 92%; or such as about 87% to about 91%) at about 24 months. In a further aspect, the adjuvant EGFR TKI treatment provides a probability of DFS of about 80% (such as 77%, 78%, 79%, 80%, 81% or 82%) at about 36 months. In a further aspect, the adjuvant EGFR TKI treatment provides a probability of DFS of about 75% to about 85% (such as about 77% to about 82%; or such as about 78% to about 80%) at about 36 months.

In an aspect, the adjuvant EGFR TKI treatment provides an increase in the probability of DFS of at least about 30% (such as at least about 28%, 29%, 30%, 31% or 32%), at about 24 months. In a further aspect, the adjuvant EGFR TKI treatment provides an increase in the probability of DFS of at least about 28% to about 32% (such as at least about 29% to about 31%), at about 24 months. In a further aspect, the adjuvant EGFR TKI treatment provides an increase in the probability of DFS of at least about 30% (such as at least about 28%, 29%, 30%, 31% or 32%), at about 36 months. In a further aspect, the adjuvant EGFR TKI treatment provides an increase in the probability of DFS of at least about 28% to about 32% (such as at least about 29% to about 31%), at about 36 months.

In an aspect, the adjuvant EGFR TKI treatment provides a median DFS of at least about 48 months. In a further aspect, the adjuvant EGFR TKI treatment provides a median DFS of at least about 54 months. In a further aspect, the adjuvant EGFR TKI treatment provides a median DFS of at least about 60 months. In a further aspect, the adjuvant EGFR TKI treatment provides a median DFS of at least about 66 months. In a further aspect, the adjuvant EGFR TKI treatment provides a median DFS of at least about 72 months. TFST is defined as the time from the date of randomisation (or initiation of therapy) to the earlier of the date of anti-cancer therapy start date following discontinuation of the adjuvant EGFR TKI treatment, or death.

In an aspect, the adjuvant EGFR TKI treatment provides a TFST of at least about 12 months, such as at least about 24 months, such as at least about 36 months, such as at least about 48 months, or such as at least about 60 months.

OS is the time from randomisation (or initiation of treatment) to the date of death (from any cause).

In an aspect, the adjuvant EGFR TKI treatment provides a median OS of at least about 60 months. In a further aspect, the adjuvant EGFR TKI treatment provides a median OS of at least about 72 months. In a further aspect, the adjuvant EGFR TKI treatment provides a median OS of greater than at least about 84 months. In a further aspect, the adjuvant EGFR TKI treatment provides a median OS of greater than at least about 96 months.

Patients with locally-advanced or metastatic EGFR mutation-positive NSCLC who receive an EGFR TKI for use according to the methods set out in this specification may have been classified post-operatively as having stage IB, II or IIIA disease on the basis of pathologic criteria. Staging may be determined according to the TNM (Tumour, Node, Metastasis) staging system for lung cancer (AJCC Cancer Staging Manual, 7th edition, Springer, New York).

In an aspect, the patient is a patient classified with stage IB, II or IIIA epidermal growth factor receptor-mutation-positive (EGFRm) non-small cell lung cancer (NSCLC) following complete tumour resection. In an aspect, the patient is a patient classified with stage II or IIIA epidermal growth factor receptor-mutation-positive (EGFRm) non-small cell lung cancer (NSCLC) following complete tumour resection. In an aspect, the patient is a patient classified with stage IB epidermal growth factor receptor-mutation-positive (EGFRm) non-small cell lung cancer (NSCLC) following complete tumour resection. In an aspect, the patient is a patient classified with stage II epidermal growth factor receptor-mutation-positive (EGFRm) non-small cell lung cancer (NSCLC) following complete tumour resection. In an aspect, the patient is a patient classified with stage IIIA epidermal growth factor receptor-mutation-positive (EGFRm) non-small cell lung cancer (NSCLC) following complete tumour resection.

In an aspect, the patient has received adjuvant chemotherapy. In an aspect, the patient has not received adjuvant chemotherapy.

## EXAMPLES

*A Phase III, Double-blind, Randomized, Placebo-Controlled Multi-centre, study to assess the efficacy and safety of AZD9291 versus Placebo, in Patients with Epidermal Growth Factor Receptor Mutation Positive Stage IB-IIIA Non-small Cell Lung Carcinoma, following Complete Tumour Resection With or Without Adjuvant Chemotherapy (ADAURA)*

ADAURA (NCT02511106; Clinical Lung Cancer [2018], Vol. 19, No. 4, e533-36) is a phase 3 double-blind, randomized, placebo-controlled, study to assess the efficacy and safety of AZD9291 versus placebo in patients with stage

IB-IIIA non-small cell lung cancer (NSCLC) with centrally confirmed, most common sensitising EGFR mutations (Ex19Del and L858R) either alone or in combination with other EGFR mutations as confirmed by a central test, who have had complete tumour resection, with or without postoperative adjuvant chemotherapy. Adjuvant chemotherapy should have consisted of a platinum based doublet given for a maximum of 4 cycles.

**Overview of Study Design**

Patients were randomised 1:1, to receive either AZD9291 or placebo. Patients had sufficiently recovered from surgery and completed any standard of care adjuvant chemotherapy prior to randomization. Patients were randomised within 10 weeks following complete surgical resection if adjuvant chemotherapy was not administered, within 26 weeks following surgery if adjuvant chemotherapy was administered.

Accounting for patients who are found to have wild type EGFR, sample attrition and 10% screen fail rate for other reasons, it was estimated that approximately 3200 patients would be screened to randomize approximately 700 patients. Approximately 60% of patients were recruited from Asia and 40% from non-Asian countries. The proportion of patients randomized with stage 1B cancer was approximately 30% and the proportion of patients randomized with stage II-IIIA cancer was approximately 70%. Patients were stratified at randomization by stage (IB vs II vs IIIA), mutation type (Ex19Del/L858R either alone or in combination with other EGFR mutations) as confirmed by a central laboratory using a tissue based test, and race (Asian/ non-Asian).

Following complete resection all patients were required to have a baseline CT scan (chest and abdomen including liver and adrenal glands) within 28 days prior to treatment initiation to confirm that disease is not present.

Patients undergo safety assessments at baseline, 2 weeks, 4 weeks, 12 weeks, and every 12 weeks until treatment is completed or discontinued. All study patients must have a 28 day follow up visit after treatment is stopped. Patients were treated for a maximum of 3 years or until disease recurrence or other discontinuation criteria was met.

Patients undergo regular CT scan (chest and abdomen including liver and adrenal glands) with additional anatomy imaging, as indicated by signs and symptoms of the patient, for disease recurrence. Disease free survival (DFS) shall be measured from the date of randomization until date of disease recurrence or death (from any cause) in the absence of recurrence by investigational site assessment.

Patients will be followed for disease recurrence at 12 weeks, 24 weeks, and then every 24 weeks until 5 years (taken to be 264 weeks) and yearly thereafter. Following disease recurrence, patients will be followed for overall survival (OS) every 24 weeks until 5 years (taken to be 264 weeks), then yearly thereafter.

At disease recurrence, patients will be restaged and all sites of NSCLC relapse will be recorded. Treatments received by the patient after relapse will be determined by the physician. Post recurrence cancer treatments and procedures will be recorded.

An Independent Data Monitoring Committee (IDMC) was convened and met approximately every 6 months for the first 2 years from the first patient randomized and approximately yearly thereafter. The IDMC will review safety assessments and make recommendations to continue, amend, or stop the study based on safety findings. Serious adverse events, adverse events, and other safety data will be reviewed, and individual and aggregated safety data will be evaluated by the IDMC.

Patients will be initially followed as per the study protocol until data cut-off for the primary analysis, estimated as 68-70 months following first subject randomised, based on a 28 month recruitment period. If there are meaningfully less than 70 DFS events in the IB population at the time of the primary analysis, further follow up of all patients according to the same study plan will be performed until the 'IB analysis'.

Following the primary analysis (or 'IB analysis' if required), patients will be followed-up for survival according to a reduced study plan (OS extension period) until the data cut-off for the extended OS analysis, which will occur approximately one year post primary analysis data cut-off date. Alternatively (or additionally), patients will be followed-up for survival 5 years post last subject in (LSI), which will occur approximately two years post primary analysis data cut-off date. This extended OS analysis will not be driven by the number of events, and therefore the results will be treated as exploratory only. Should there be any remaining patients on study drug at the time of data cut-off for the extended OS analysis, they will be able to continue study treatment until completion or a treatment discontinuation criterion is met.

Following the extended OS analysis, the ADAURA study will be closed, and the collection of survival, cancer therapy and safety data for globally recruited patients no longer on study treatment will stop entirely. Any patients still on study drug will be managed outside of the study.

**Target patient population**

Male and female patients aged 18 years and over (patients from Japan/Taiwan aged at least 20 years) with histologically confirmed diagnosis of primary non-squamous non-small cell carcinoma of the lung, with complete surgical resection of the primary tumour, and who were classified post-operatively as stage IB, II or IIIA on the basis of pathologic

criteria were eligible for this study (stage IB patients were excluded in Japan). Recruitment of patients with stage IB disease was closed when approximately 210 (30%) had been randomized and recruitment of patients with stage II-IIIA disease was closed when approximately 470 (70%) had been randomized. Patients were confirmed to have a tumour that harbours one of the most common EGFR mutations known to be associated with EGFR-TKI sensitivity (Ex19Del; L858R) either alone or in combination with other EGFR mutations as confirmed by a central test.

Patients may have received prior adjuvant platinum doublet chemotherapy for a maximum of 4 cycles in accordance with standard of care but must not have received prior radiotherapy. Pre-operative (neo-adjuvant) platinum based or other chemotherapy was not allowed.

**Duration of treatment**

Treatment with AZD9291 (80 mg once daily) or placebo commenced following randomization. Patients will continue on randomised treatment until recurrence of disease, treatment discontinuation criterion is met or treatment is completed. The maximum treatment duration period is 3 years (156 weeks).

**Investigational product, dosage and mode of administration**

AZD9291 is an oral, potent, selective, central nervous system (CNS) active, irreversible epidermal growth factor receptor (EGFR) tyrosine kinase inhibitor (TKI) effective against both EGFR-TKI sensitising and resistance mutations in NSCLC with a significant selectivity margin over wild-type EGFR. AZD9291 (80 mg orally, once daily) or matching placebo, in accordance with the randomization schedule, was administered.

**Key inclusion criteria**

a) Histologically confirmed diagnosis of primary non small lung cancer (NSCLC) on predominantly non-squamous histology.

b) Patients must be classified post-operatively as stage IB, II or IIIA on the basis of pathologic criteria. Staging will be according to the TNM (Tumour, Node, Metastasis) staging system for lung cancer (7th edition).

c) Confirmation by the central laboratory that the tumour harbours one of the 2 common EGFR mutations known to be associated with EGFR-TKI sensitivity (Ex19del, L858R), either alone or in combination with other EGFR mutations including T790M.

d) Complete surgical resection of the primary NSCLC is mandatory. All gross disease must have been removed at the end of surgery. All surgical margins of resection must be negative for tumour. Resection may be accomplished by open or Video Associated Thoracic Surgery (VATS) techniques.

e) Complete recovery from surgery and standard post-operative therapy (if applicable) at the time of randomization. Treatment cannot commence within 4 weeks following surgery. No more than 10 weeks may have elapsed between surgery and randomization for patients who have not received adjuvant chemotherapy; no more than 26 weeks may have elapsed between surgery and randomization for patients who received adjuvant chemotherapy. Complete post-operative wound healing must have occurred following any surgery. For patients who received post-operative adjuvant platinum-based chemotherapy, a minimum of 2 weeks must have elapsed (but no more than 10 weeks) from the last administered dose of chemotherapy to the date of randomization. Patients must have recovered from all toxicities of prior therapy greater than CTCAE Grade 1 at the time of starting study treatment with the exception of alopecia and Grade 2 prior platinum therapy related neuropathy.

f) World Health Organization Performance Status of 0 to 1.

**Key exclusion criteria**

a) Treatment with any of the following: Pre-operative or post-operative or planned radiation therapy for the current lung cancer; Pre-operative (neo-adjuvant) platinum based or other chemotherapy; Any prior anticancer therapy, including

investigational therapy, for treatment of NSCLC other than standard platinum based doublet post-operative adjuvant chemotherapy; Prior treatment with neoadjuvant or adjuvant EGFR-TKI; Major surgery (including primary tumour surgery, excluding placement of vascular access) within 4 weeks of the first dose of study drug; Patients currently receiving (or unable to stop use prior to receiving the first dose of study treatment) medications or herbal supplements known to be potent inducers of CYP3A4 (at least 3 week prior); Treatment with an investigational drug within five half-lives of the compound or any of its related material, if known.

b) Patients who have had only segmentectomies or wedge resections.

c) History of other malignancies, except: adequately treated non-melanoma skin cancer, curatively treated in-situ cancer, or other solid tumours curatively treated with no evidence of disease for > 5 years following the end of treatment and which, in the opinion of the treating physician, do not have a substantial risk of recurrence of the prior malignancy.

d) Any unresolved toxicities from prior therapy greater than CTCAE Grade 1 at the time of starting study treatment with the exception of alopecia and Grade 2, prior platinum-therapy related neuropathy.

e) Any evidence of severe or uncontrolled systemic diseases, including uncontrolled hypertension and active bleeding diatheses, which in the Investigator's opinion makes it undesirable for the patient to participate in the trial or which would jeopardise compliance with the protocol; or active infection including hepatitis B, hepatitis C and human immunodeficiency virus (HIV). Active infection will include any patients receiving intravenous treatment for infection; active hepatitis B infection will, at a minimum, include all patients who are hepatitis B surface antigen positive (HbsAg positive) based on serology assessment.

f) Refractory nausea and vomiting, chronic gastrointestinal diseases, inability to swallow the formulated product, or previous significant bowel resection that would preclude adequate absorption of AZD9291.

g) Any of the following cadiac criteria: Mean resting corrected QT interval (QTc) >470 msec, obtained from 3 ECGs, using the screening clinic ECG machine-derived QTcF value; Any clinically important abnormalities in rhythm, conduction, or morphology of resting ECG, e.g., complete left bundle branch block, third-degree heart block, second degree heart block; Any factors that increase the risk of QTc prolongation or risk of arrhythmic events such as heart failure, hypokalaemia, congenital long QT syndrome, family history of long QT syndrome, or unexplained sudden death under 40 years of age in first-degree relatives or any concomitant medication known to prolong the QT interval.

h) Past medical history of ILD, drug-induced ILD, radiation pneumonitis which required steroid treatment, or any evidence of clinically active ILD.

i) Inadequate bone marrow reserve or organ function as demonstrated by any of the following laboratory values: Absolute neutrophil count <1.5 x 109/L; Platelet count <100 x 109/L; Haemoglobin <90 g/L; p- Alanine aminotransferase (ALT) >2.5x the upper limit of normal (ULN); Aspartate aminotransferase (AST) >2.5xULN; Total bilirubin >1.5xULN or >3xULN in the presence of documented Gilbert's Syndrome (unconjugated hyperbilirubinaemia); Creatinine >1.5xULN concurrent with creatinine clearance <50 mL/min (measured or calculated by Cockcroft and Gault equation); confirmation of creatinine clearance is only required when creatinine is >1.5xULN.

**Efficacy Assessments**

<u>Disease Free Survival (DFS)</u>

Disease-free survival is the primary endpoint in this study and is defined as the time from the date of randomization until the date of disease recurrence or death from any cause in the absence of disease recurrence. Disease recurrence is defined as evidence of disease recurrence on CT or MRI scan and/or pathological disease on biopsy by investigational site assessment. The primary population is the stage II/IIIA patients.

DFS rate at 2, 3, 4 and 5 years is defined as the proportion of patients alive and disease free at 2, 3, 4 and 5 years, respectively, estimated from Kaplan Meier plots of the primary endpoint of DFS at the time of the primary analysis.

DFS in the subset of patients with stage II-IIIA cancer will be analysed using a log rank test stratified by stage (II, IIIA), mutation type (Ex19Del, L858R either alone or in combination with other EGFR mutations) and race (Asian, Non-Asian) for the generation of the p-value and using the Breslow approach for handling ties. DFS in the overall population will be analysed using a log rank test stratified by stage (IB, II, IIIA), mutation status (Ex19Del, L858R either alone or in

combination with other EGFR mutations as confirmed by a central test) and race (Asian, Non-Asian) for the generation of the p-value and using the Breslow approach for handling ties. The hazard ratio and confidence interval will be obtained directly from the U and V statistics as follows (Berry et al 1991, Statistics in Medicine, Volume 10, pp. 749-55; Selke and Siegmund 1983, Biometrika, Volume 70, pp. 315-26):

$$HR = \exp(U/V)$$

$$95\%\ CI\ for\ HR = (\exp\{U/V - 1.96/\sqrt{V}\}, \exp\{U/V + 1.96\sqrt{V}\})$$

Where $U = \Sigma_i(d_{1i}-e_{1i})$ is the log-rank test statistic (with $d_{1i}$ and $e_{1i}$ the observed and expected events in group 1) and $\sqrt{V}$ the standard deviation of the log-rank test statistic obtained from the LIFETEST procedure with a STRATA term for the stratification variables. HR is hazard ratio and CI is confidence interval.

A Kaplan-Meier (KM) plot of DFS will be presented by treatment group.

Overall Survival (OS)

Overall survival is defined as the time from randomization to the date of death (from any cause).

Following randomization, patients will be followed up for survival every 24 weeks for 5 years (264 weeks) and then yearly thereafter, until the study is closed.

OS rate at 2, 3, 4, and 5 years is defined as the proportion of patients alive at 2, 3, 4, and 5 years respectively, estimated from a Kaplan Meier plot of OS at the time of the primary analysis.

OS data will be analysed at the time of primary analysis using a log rank test stratified by stage (IB, II, IIIA), mutation type (Ex19Del, L858R either alone or in combination with other EGFR mutations) and race (Asian, Non-Asian) for the generation of the p-value and using the Breslow approach for handling ties. The hazard ratio and confidence interval will be obtained directly from the U and V statistics, as described above (provided there are sufficient events available for a meaningful analysis (>20 deaths), if not descriptive summaries will be provided).

Time to first subsequent therapy or death

Time to first subsequent therapy (TFST) or death is defined as the time from the date of randomization to the earlier of the date of anti-cancer therapy start date following study drug discontinuation, or death. Any patient not known to have had a subsequent therapy or not known to have died at the time of the analysis will be censored at the last known time to have not received subsequent therapy; i.e., the last follow-up visit where this was confirmed.

**Results**

ADAURA was unblinded early following a recommendation from an Independent Data Monitoring Committee (IDMC) based on its determination of overwhelming efficacy. The results presented herein are derived from an analysis conducted after data cut off in January 2020. DCO had originally been expected in February 2022.

KM plots of DFS for the overall patient group, patients with stage II-IIIA disease, patients with stage IB disease, patients with stage II disease, patients with stage IIIA disease and patients who received and did not receive adjuvant chemotherapy are shown in Figures 1-5, 7 and 8 respectively. The data are summarised in Tables 1 and 2 below.

The key findings were:

- There was an 83% reduction in the risk of disease recurrence or death with osimertinib vs placebo (DFS HR=0.17 (0.12, 0.23); p<0.0001) in the primary population of stage II/IIIA patients
- In the overall population, there was a 79% reduction in the risk of disease recurrence or death with osimertinib vs placebo (DFS HR 0.21 [95% CI 0.16, 0.28]; p<0.0001)
- In the overall population, osimertinib vs placebo DFS rates at 2 years were 89 vs 53%, respectively
- A consistent improvement in DFS was seen regardless of whether patients received prior adjuvant chemotherapy
- Immature OS (5% maturity) showed a trend towards survival favouring osimertinib:
    - Stage II / IIIA patient population: osimertinib: 8 deaths (3%), placebo: 17 deaths (7%)

Table 1

| | Stage II-IIIA<br>n(o)=233<br>n(p)=237 | Stage IB/II/IIIA<br>n(o)=339<br>n(p)=343 | Adjuvant chemotherapy<br>n(o)=187<br>n(p)=191 | No adjuvant chemotherapy<br>n(o)=152<br>n(p)=152 |
|---|---|---|---|---|
| Median DFS, months (95%CI)<br>**Osimertinib**<br>*Placebo* | **NR (38.8, NC)**<br>*20.4 (16.6, 24.5)* | **NR (NC. NC)**<br>*28.1 (22.1, 35.8)* | **NR (38.8, NC)**<br>*22.3 (17.5, 35.0)* | **NR (NC, NC)**<br>*33.1 (22.1, NC)* |
| HR (95% CI) | 0.17 (0.12, 0.23); p<0.0001 | 0.21 (0.16, 0.28); p<0.0001 | 0.17 (0.11, 0.29) | 0.17 (0.13, 0.38) |
| Maturity, % | 33 | 29 | 30 | 27 |
| 2 year DFS rate, % (95%CI)<br>**Osimertinib**<br>*Placebo* | **89.5 (84.0, 93.2)**<br>*43.8 (36.7, 50.8)* | **88.8 (84.1, 92.1)**<br>*52.9 (46.9, 58.6)* | **88.3 (81.8, 92.6)**<br>*49.3 (41.1, 57.1)* | **89.2 (81.6, 93.8)**<br>*57.3 (48.2, 65.4)* |
| Median follow up, months<br>**Osimertinib**<br>*Placebo* | **22.1**<br>*15.0* | **22.1**<br>*16.6* | **22.1**<br>*16.6* | **22.1**<br>*18.2* |
| DFS, disease free survival; n, number of patients receiving osimertinib (o) or placebo (p); HR, hazard ratio; CI, confidence interval; NR, not reached; NC, not calculated; p, probability value | | | | |

Table 2

| | Stage IB<br>n(o)=106<br>n(p)=106 | Stage II<br>n(o)=118<br>n(p)=118 | Stage IIIA<br>n(o)=115<br>n(p)=119 |
|---|---|---|---|
| Median DFS, months (95%CI)<br>**Osimertinib**<br>*Placebo* | **NR (NC, NC)**<br>*48.2 (NC, NC)* | **NR (NC, NC)**<br>*28.1 (20.4, NC)* | **38.8 (38.6, NC)**<br>*12.9 (10.9, 18.3)* |
| HR (95% CI) | 0.50 (0.27, 0.94) | 0.17 (0.08, 0.31) | 0.12 (0.07, 0.20) |
| Maturity, % | 19 | 27 | 40 |
| 2 year DFS rate, % (95%CI)<br>**Osimertinib**<br>*Placebo* | 87 (77, 93)<br>73 (62, 81) | 91 (82, 95)<br>56 (45, 65) | 88 (79, 94)<br>32 (23, 42) |
| Median follow up, months<br>**Osimertinib**<br>*Placebo* | **22.1**<br>*22.0* | **22.1**<br>*16.6* | **22.1**<br>*11.1* |
| DFS, disease free survival; n, number of patients receiving osimertinib (o) or placebo (p); HR, hazard ratio; CI, confidence interval; NR, not reached; NC, not calculated; p, probability value | | | |

## Claims

1. An epidermal growth factor receptor (EGFR) tyrosine kinase inhibitor (TKI) for use in the adjuvant treatment after tumour resection of a patient with epidermal growth factor receptor-mutation-positive (EGFRm) non-small cell lung cancer (NSCLC) , wherein the EGFR TKI is either a second-generation or third-generation EGFR TKI.

2. An EGFR TKI for use according to claim 1, wherein the patient has been classified with stage II or IIIA EGFRm NSCLC.

**3.** An EGFR TKI for use according to claim 1 or claim 2, wherein the patient has received adjuvant chemotherapy.

**4.** An EGFR TKI for use according to any one of claims 1 to 3, wherein the EGFRm NSCLC comprises activating mutations in EGFR selected from exon 19 deletions or exon 21 L858R substitution mutations.

**5.** An EGFR TKI for use according to any one of claims 1 to 4, wherein the adjuvant EGFR TKI treatment provides improved disease free survival (DFS).

**6.** An EGFR TKI for use according to any one of claims 1 to 5, wherein the adjuvant EGFR TKI treatment provides a probability of DFS of about 85% to about 95% at about 24 months.

**7.** An EGFR TKI for use according to any one of claims 1 to 6, wherein the EGFR TKI is a third-generation EGFR TKI.

**8.** An EGFR TKI for use according to any one of claims 1 to 7, wherein the EGFR TKI is a third-generation EGFR TKI of Formula (I):

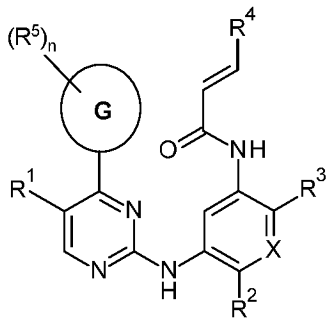

**(I)**

wherein:

**G** is selected from 4,5,6,7-tetrahydropyrazolo[1,5-*a*]pyridin-3-yl, indol-3-yl, indazol-1-yl, 3,4-dihydro-1H-[1,4] oxazino[4,3-a]indol-10-yl, 6,7,8,9-tetrahydropyrido[1,2-a]indol-10-yl, 5,6-dihydro-4H-pyrrolo[3,2,1-ij]quinolin-1-yl, pyrrolo[3,2-b]pyridin-3-yl and pyrazolo[1,5-*a*]pyridin-3-yl;
$\mathbf{R^1}$ is selected from hydrogen, fluoro, chloro, methyl and cyano;
$\mathbf{R^2}$ is selected from methoxy, trifluoromethoxy, ethoxy, 2,2,2-trifluoroethoxy and methyl;
$\mathbf{R^3}$ is selected from (3*R*)-3-(dimethylamino)pyrrolidin-1-yl, (3*S*)-3-(dimethyl-amino)pyrrolidin-1-yl, 3-(dimethylamino)azetidin-1-yl, [2-(dimethylamino)ethyl]-(methyl)amino, [2-(methylamino)ethyl](methyl)amino, 2-(dimethylamino)ethoxy, 2-(methylamino)ethoxy, 5-methyl-2,5-diazaspiro[3.4]oct-2-yl, (3a*R*,6a*R*)-5-methylhexa-hydropyrrolo[3,4-b]pyrrol-1(2*H*)-yl, 1-methyl-1,2,3,6-tetrahydropyridin-4-yl, 4-methylpiperizin-1-yl, 4-[2-(dimethylamino)-2-oxoethyl]piperazin-1-yl, methyl[2-(4-methylpiperazin-1-yl)ethyl]amino, methyl[2-(morpholin-4-yl)ethyl] amino, 1-amino-1,2,3,6-tetrahydropyridin-4-yl and 4-[(2*S*)-2-aminopropanoyl]piperazin-1-yl;
$\mathbf{R^4}$ is selected from hydrogen, 1-piperidinomethyl and N,N-dimethylaminomethyl;
$\mathbf{R^5}$ is independently selected from methyl, ethyl, propyl, 2,2-difluoroethyl, 2,2,2-trifluoroethyl, fluoro, chloro and cyclopropyl;
**X is** CH or N; and
**n** is 0, 1 or 2;
or a pharmaceutically acceptable salt thereof.

**9.** An EGFR TKI for use according to any one of claims 1 to 7, wherein the third-generation EGFR TKI is selected from the group consisting of osimertinib or a pharmaceutically acceptable salt thereof, AZD3759 or a pharmaceutically acceptable salt thereof, lazertinib or a pharmaceutically acceptable salt thereof, abivertinib or a pharmaceutically acceptable salt thereof, alflutinib or a pharmaceutically acceptable salt thereof, CK-101 or a pharmaceutically acceptable salt thereof, HS-10296 or a pharmaceutically acceptable salt thereof and BPI-7711 or a pharmaceutically acceptable salt thereof.

**10.** An EGFR TKI for use according to any one of claims 1 to 9, wherein the third generation EGFR TKI is osimertinib, or a pharmaceutically acceptable salt thereof.

11. An EGFR TKI for use according to claim 10, wherein the osimertinib, or a pharmaceutically acceptable salt thereof, is administered once-daily.

12. An EGFR TKI for use according to either claim 10 or claim 11, wherein the osimertinib, or a pharmaceutically acceptable salt thereof, is administered in tablet form.

13. An EGFR TKI for use according to any one of claims 10 to 12, wherein the osimertinib, or a pharmaceutically acceptable salt thereof, is osimertinib mesylate.

14. An EGFR TKI for use according to any one of claims 1 to 6, wherein the EGFR TKI is a second-generation EGFR TKI selected from afatinib and dacomitinib.

**Patentansprüche**

1. Tyrosinkinaseinhibitor (TKI) für den epidermalen Wachstumsfaktorrezeptor (EGFR) zur Verwendung bei der adjuvanten Behandlung nach Tumorresektion eines Patienten mit epidermalem Wachstumsfaktorrezeptor-Mutations-positivem (EGFRm) nicht-kleinzelligem Lungenkrebs (NSCLC), wobei der EGFR-TKI entweder ein EGFR-TKI der zweiten oder dritten Generation ist.

2. EGFR-TKI zur Verwendung nach Anspruch 1, wobei der Patient mit EGFRm NSCLC im Stadium II oder IIIA klassifiziert wurde.

3. EGFR-TKI zur Verwendung nach Anspruch 1 oder 2, wobei der Patient eine adjuvante Chemotherapie empfangen hat.

4. EGFR-TKI zur Verwendung nach einem der Ansprüche 1 bis 3, wobei der EGFRm NSCLC aktivierende Mutationen im EGFR umfasst, die aus Exon-19-Deletionen oder Exon-21-L858R-Substitutionsmutationen ausgewählt sind.

5. EGFR-TKI zur Verwendung nach einem der Ansprüche 1 bis 4, wobei die adjuvante EGFR-TKI-Behandlung ein verbessertes krankheitsfreies Überleben (DFS) bereitstellt.

6. EGFR-TKI zur Verwendung nach einem der Ansprüche 1 bis 5, wobei die adjuvante EGFR-TKI-Behandlung eine Wahrscheinlichkeit des DFS von etwa 85 % bis etwa 95 % nach etwa 24 Monaten bereitstellt.

7. EGFR-TKI zur Verwendung nach einem der Ansprüche 1 bis 6, wobei der EGFR-TKI ein EGFR-TKI der dritten Generation ist.

8. EGFR-TKI zur Verwendung nach einem der Ansprüche 1 bis 7, wobei der EGFR-TKI ein EGFR-TKI der dritten Generation ist, der die Formel (I) aufweist:

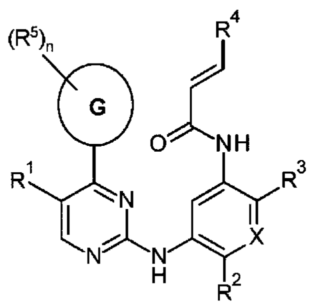

(I)

wobei:

**G** ausgewählt ist aus 4,5,6,7-Tetrahydropyrazolo[1,5-$\alpha$]pyridin-3-yl, Indol-3-yl, Indazol-1-yl, 3,4-Dihydro-1H-[1,4]oxazino[4,3-a]indol-10-yl, 6,7,8,9-Tetrahydropyrido[1,2-a]indol-10-yl, 5,6-Dihydro-4H-pyrrolo[3,2,1-ij]chinolin-1-yl, Pyrrolo[3,2-b]pyridin-3-yl und Pyrazolo[1,5-$\alpha$]pyridin-3-yl;
**$R^1$** ausgewählt ist aus Wasserstoff, Fluor, Chlor, Methyl und Cyano;

**$R^2$** ausgewählt ist aus Methoxy, Trifluormethoxy, Ethoxy, 2,2,2-Trifluorethoxy und Methyl;
**$R^3$** ausgewählt ist aus (3*R*)-3-(Dimethylamino)pyrrolidin-1-yl, (3*S*)-3-(Dimethyl-amino)pyrrolidin-1-yl, 3-(Dimethylamino)azetidin-1-yl, [2-(Dimethylamino)ethyl]-(methyl)amino, [2-(Methylamino)ethyl](methyl)amino, 2-(Dimethylamino)ethoxy, 2-(Methylamino)ethoxy, 5-Methyl-2,5-diazaspiro[3.4]oct-2-yl, (3a*R*,6a*R*)-5-Methylhexahydro-pyrrolo[3,4-*b*]pyrrol-1(2*H*)-yl, 1-Methyl-1,2,3,6-tetrahydropyridin-4-yl, 4-Methylpiperizin-1-yl, 4-[2-(Dimethylamino)-2-oxoethyl]piperazin-1-yl, Methyl[2-(4-methylpiperazin-1-yl)ethyl]amino, Methyl[2-(morpholin-4-yl) ethyl]amino, 1-Amino-1,2,3,6-tetrahydropyridin-4-yl und 4-[(2*S*)-2-aminopropanoyl]piperazin-1-yl;
**$R^4$** ausgewählt ist aus Wasserstoff, 1-Piperidinomethyl und N,N-Dimethylaminomethyl;
**$R^5$** unabhängig ausgewählt ist aus Methyl, Ethyl, Propyl, 2,2-Difluorethyl, 2,2,2-Trifluorethyl, Fluor, Chlor und Cyclopropyl;
**X** CH oder N ist; und
**n** 0, 1 oder 2 ist;
oder ein pharmazeutisch verträgliches Salz davon.

**9.** EGFR-TKI zur Verwendung nach einem der Ansprüche 1 bis 7, wobei der EGFR-TKI der dritten Generation ausgewählt ist aus der Gruppe, bestehend aus Osimertinib oder einem pharmazeutisch verträglichen Salz davon, AZD3759 oder einem pharmazeutisch verträglichen Salz davon, Lazertinib oder einem pharmazeutisch verträglichen Salz davon, Abivertinib oder einem pharmazeutisch verträglichen Salz davon, Alflutinib oder einem pharmazeutisch verträglichen Salz davon, CK-101 oder einem pharmazeutisch verträglichen Salz davon, HS-10296 oder einem pharmazeutisch verträglichen Salz davon und BPI-7711 oder einem pharmazeutisch verträglichen Salz davon.

**10.** EGFR-TKI zur Verwendung nach einem der Ansprüche 1 bis 9, wobei der EGFR-TKI der dritten Generation Osimertinib oder ein pharmazeutisch verträgliches Salz davon ist.

**11.** EGFR-TKI zur Verwendung nach Anspruch 10, wobei das Osimertinib oder ein pharmazeutisch verträgliches Salz davon einmal täglich verabreicht wird.

**12.** EGFR-TKI zur Verwendung nach Anspruch 10 oder 11, wobei das Osimertinib oder ein pharmazeutisch verträgliches Salz davon in Tablettenform verabreicht wird.

**13.** EGFR-TKI zur Verwendung nach einem der Ansprüche 10 bis 12, wobei das Osimertinib oder ein pharmazeutisch verträgliches Salz davon Osimertinibmesylat ist.

**14.** EGFR-TKI zur Verwendung nach einem der Ansprüche 1 bis 6, wobei der EGFR-TKI ein EGFR-TKI der zweiten Generation ist, ausgewählt aus Afatinib und Dacomitinib.

## Revendications

**1.** Inhibiteur de la tyrosine kinase (ITK) du récepteur du facteur de croissance épidermique (EGFR) pour une utilisation dans le traitement adjuvant après résection tumorale d'un patient atteint du cancer du poumon non à petites cellules (CPNPC) positif pour la mutation du récepteur du facteur de croissance épidermique (EGFRm), dans lequel l'ITK de l'EGFR est un ITK de l'EGFR de deuxième ou de troisième génération.

**2.** ITK de l'EGFR pour une utilisation selon la revendication 1, dans lequel le patient a été classé comme présentant un CPNPC EGFRm de stade II ou IIIA.

**3.** ITK de l'EGFR pour une utilisation selon la revendication 1 ou la revendication 2, dans lequel le patient a reçu une chimiothérapie adjuvante.

**4.** ITK de l'EGFR pour une utilisation selon l'une quelconque des revendications 1 à 3, dans lequel le CPNPC EGFRm comprend des mutations activatrices dans l'EGFR choisies parmi des délétions de l'exon 19 ou des mutations de substitution L858R de l'exon 21.

**5.** ITK de l'EGFR pour une utilisation selon l'une quelconque des revendications 1 à 4, dans lequel le traitement adjuvant par ITK de l'EGFR fournit une survie sans maladie (SSM) améliorée.

**6.** ITK de l'EGFR pour une utilisation selon l'une quelconque des revendications 1 à 5, dans lequel le traitement adjuvant

par ITK de l'EGFR fournit une probabilité de SSM d'environ 85 % à environ 95 % à environ 24 mois.

7. ITK de l'EGFR pour une utilisation selon l'une quelconque des revendications 1 à 6, dans lequel l'ITK de l'EGFR est un ITK de l'EGFR de troisième génération.

8. ITK de l'EGFR pour une utilisation selon l'une quelconque des revendications 1 à 7, dans lequel l'ITK de l'EGFR est un ITK de l'EGFR de troisième génération de formule (I) :

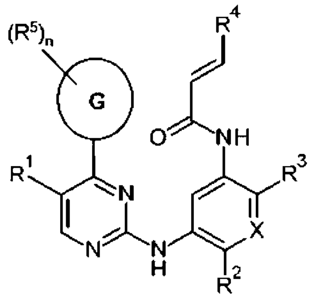

(I)

où :

**G** est choisi parmi 4,5,6,7-tétrahydropyrazolo[1,5-$\alpha$]pyridin-3-yle, indol-3-yle, indazol-1-yle, 3,4-dihydro-1H-[1,4]oxazino[4,3-a]indol-10-yle, 6,7,8,9-tétrahydropyrido[1,2-a]indol-10-yle, 5,6-dihydro-4H-pyrrolo[3,2,1-ij]quinoléin-1-yle, pyrrolo[3,2-b]pyridin-3-yle et pyrazolo[1,5-$\alpha$]pyridin-3-yle ;
**$R^1$** est choisi parmi hydrogène, fluoro, chloro, méthyle et cyano ;
**$R^2$** est choisi parmi méthoxy, trifluorométhoxy, éthoxy, 2,2,2-trifluoroéthoxy et méthyle ;
**$R^3$** est choisi parmi (3*R*)-3-(diméthylamino)pyrrolidin-1-yle, (3*S*)-3-(diméthyl-amino)pyrrolidin-1-yle, 3-(diméthylamino)azétidin-1-yle, [2-(diméthylamino)éthyl]-(méthyl)amino, [2-(méthylamino)éthyl](méthyl)amino, 2-(diméthylamino)éthoxy, 2-(méthylamino)éthoxy, 5-méthyl-2,5-diazaspiro[3.4]oct-2-yle, (3a*R*,6a*R*)-5-méthylhexahydro-pyrrolo[3,4-*b*]pyrrol-1(2*H*)-yle, 1-méthyl-1,2,3,6-tétrahydropyridin-4-yle, 4-méthylpipérizin-1-yle, 4-[2-(diméthylamino)-2-oxoéthyl]pipérazin-1-yle, méthyl[2-(4-méthylpipérazin-1-yl)éthyl]amino, méthyl[2-(morpholin-4-yl)éthyl]amino, 1-amino-1,2,3,6-tétrahydropyridin-4-yle et 4-[(2S)-2-aminopropanoyl]pipérazin-1-yle ;
**$R^4$** est choisi parmi hydrogène, 1-pipéridinométhyle et N,N-diméthylaminométhyle ;
**$R^5$** est choisi indépendamment parmi méthyle, éthyle, propyle, 2,2-difluoroéthyle, 2,2,2-trifluoroéthyle, fluoro, chloro et cyclopropyle ;
**X** est CH ou N ; et
**n** vaut 0, 1 ou 2 ;
ou un sel pharmaceutiquement acceptable de celui-ci.

9. ITK de l'EGFR pour une utilisation selon l'une quelconque des revendications 1 à 7, dans lequel l'ITK de l'EGFR de troisième génération est choisi dans le groupe constitué d'osimertinib ou un sel pharmaceutiquement acceptable de celui-ci, AZD3759 ou un sel pharmaceutiquement acceptable de celui-ci, lazertinib ou un sel pharmaceutiquement acceptable de celui-ci, abivertinib ou un sel pharmaceutiquement acceptable de celui-ci, alflutinib ou un sel pharmaceutiquement acceptable de celui-ci, CK-101 ou un sel pharmaceutiquement acceptable de celui-ci, HS-10296 ou un sel pharmaceutiquement acceptable de celui-ci et BPI-7711 ou un sel pharmaceutiquement acceptable de celui-ci.

10. ITK de l'EGFR pour une utilisation selon l'une quelconque des revendications 1 à 9, dans lequel l'ITK de l'EGFR de troisième génération est l'osimertinib, ou un sel pharmaceutiquement acceptable de celui-ci.

11. ITK de l'EGFR pour une utilisation selon la revendication 10, dans lequel l'osimertinib, ou un sel pharmaceutiquement acceptable de celui-ci, est administré une fois par jour.

12. ITK de l'EGFR pour une utilisation selon la revendication 10 ou la revendication 11, dans lequel l'osimertinib, ou un sel pharmaceutiquement acceptable de celui-ci, est administré sous forme de comprimé.

13. ITK de l'EGFR pour une utilisation selon l'une quelconque des revendications 10 à 12, dans lequel l'osimertinib, ou un sel pharmaceutiquement acceptable de celui-ci, est le mésylate d'osimertinib.

**14.** ITK de l'EGFR pour une utilisation selon l'une quelconque des revendications 1 à 6, dans lequel l'ITK de l'EGFR est un ITK de l'EGFR de deuxième génération choisi parmi l'afatinib et le dacomitinib.

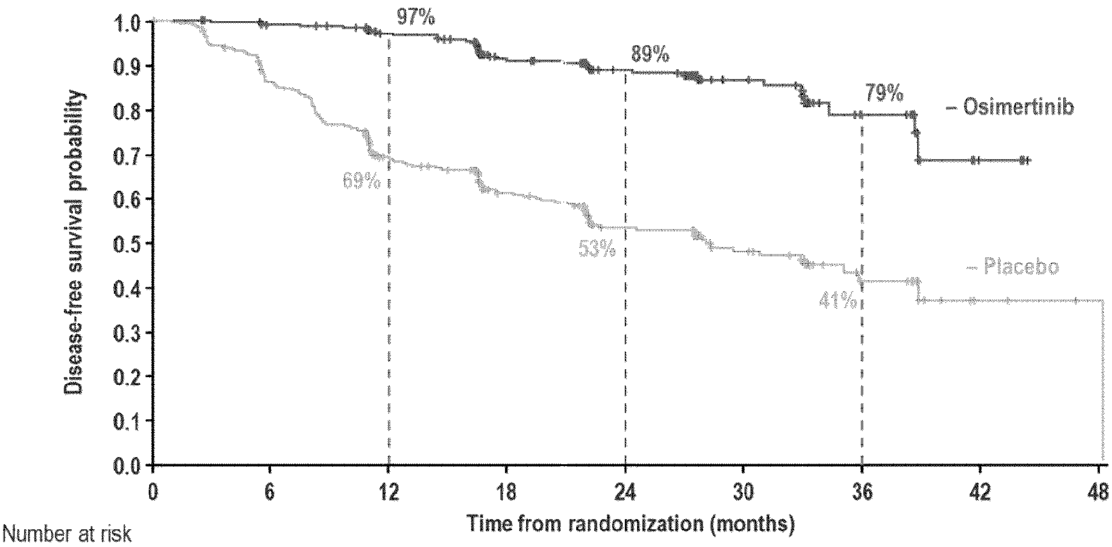


| Number at risk | | | | | | | | | |
|---|---|---|---|---|---|---|---|---|---|
| Osimertinib | 339 | 314 | 272 | 206 | 136 | 73 | 25 | 4 | 0 |
| Placebo | 343 | 288 | 209 | 149 | 87 | 53 | 20 | 3 | 1 |

**FIGURE 1**

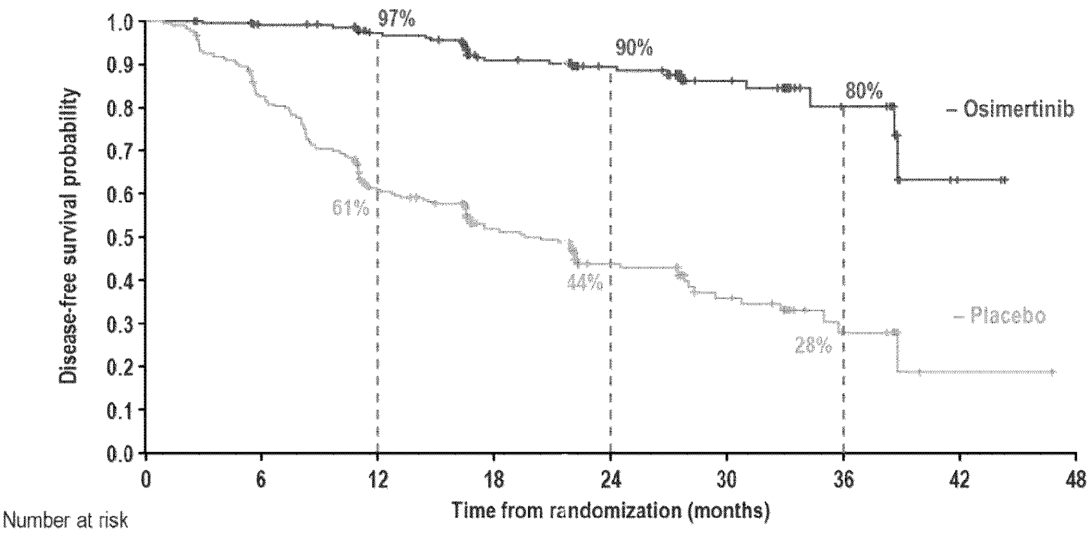


| Number at risk | | | | | | | | | |
|---|---|---|---|---|---|---|---|---|---|
| Osimertinib | 233 | 219 | 189 | 137 | 96 | 51 | 17 | 2 | 0 |
| Placebo | 237 | 190 | 128 | 82 | 51 | 27 | 9 | 1 | 0 |

**FIGURE 2**

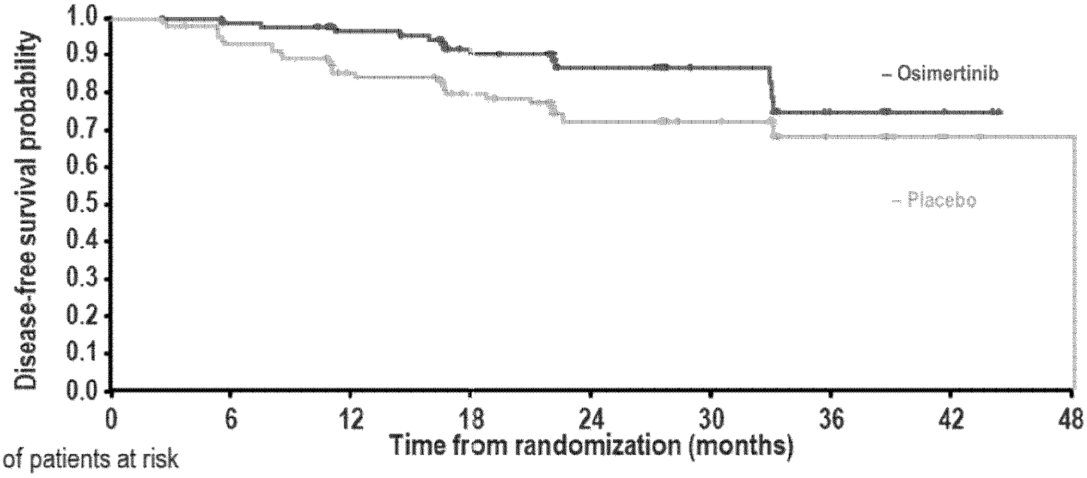


| Number of patients at risk | | | | | | | | | |
|---|---|---|---|---|---|---|---|---|---|
| Osimertinib | 106 | 95 | 83 | 69 | 40 | 22 | 8 | 2 | 0 |
| Placebo | 106 | 98 | 81 | 67 | 36 | 26 | 11 | 2 | 1 |

FIGURE 3

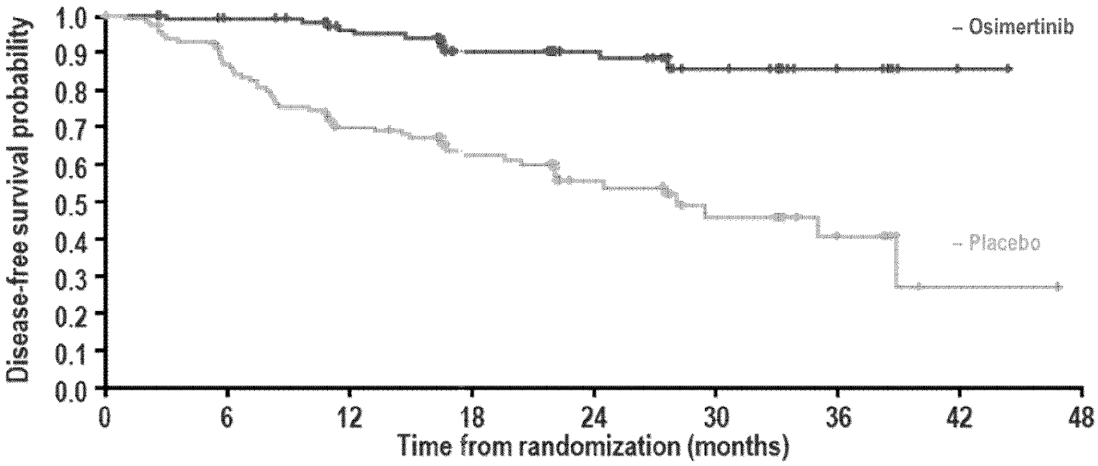


| Number at risk | | | | | | | | | |
|---|---|---|---|---|---|---|---|---|---|
| Osimertinib | 118 | 110 | 91 | 69 | 47 | 28 | 8 | 1 | 0 |
| Placebo | 118 | 99 | 74 | 49 | 31 | 15 | 7 | 1 | 0 |

FIGURE 4

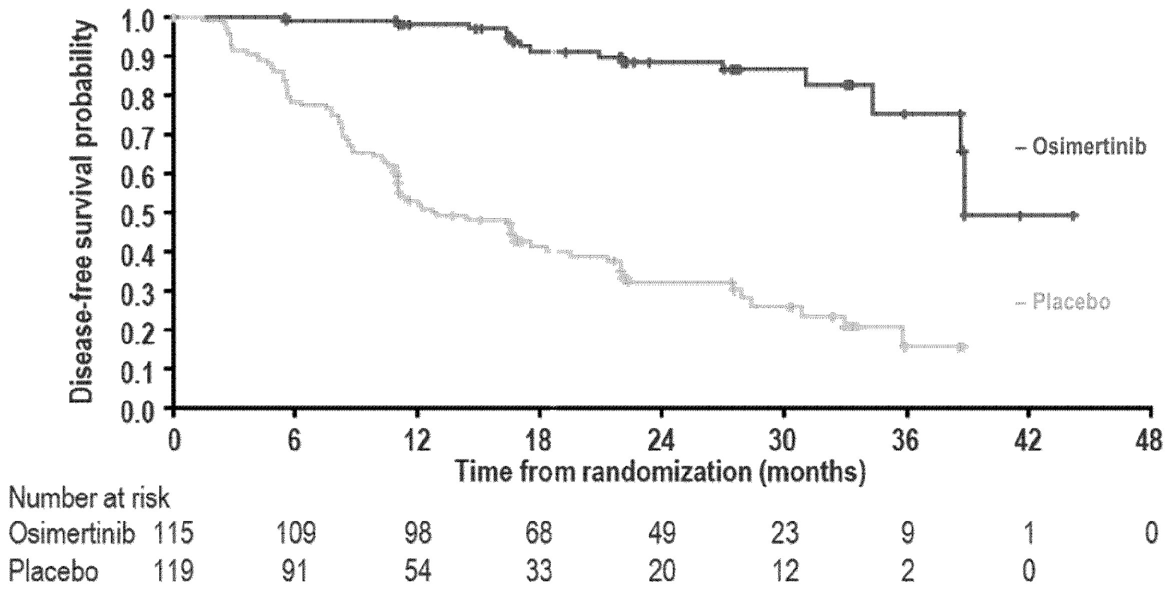


FIGURE 5

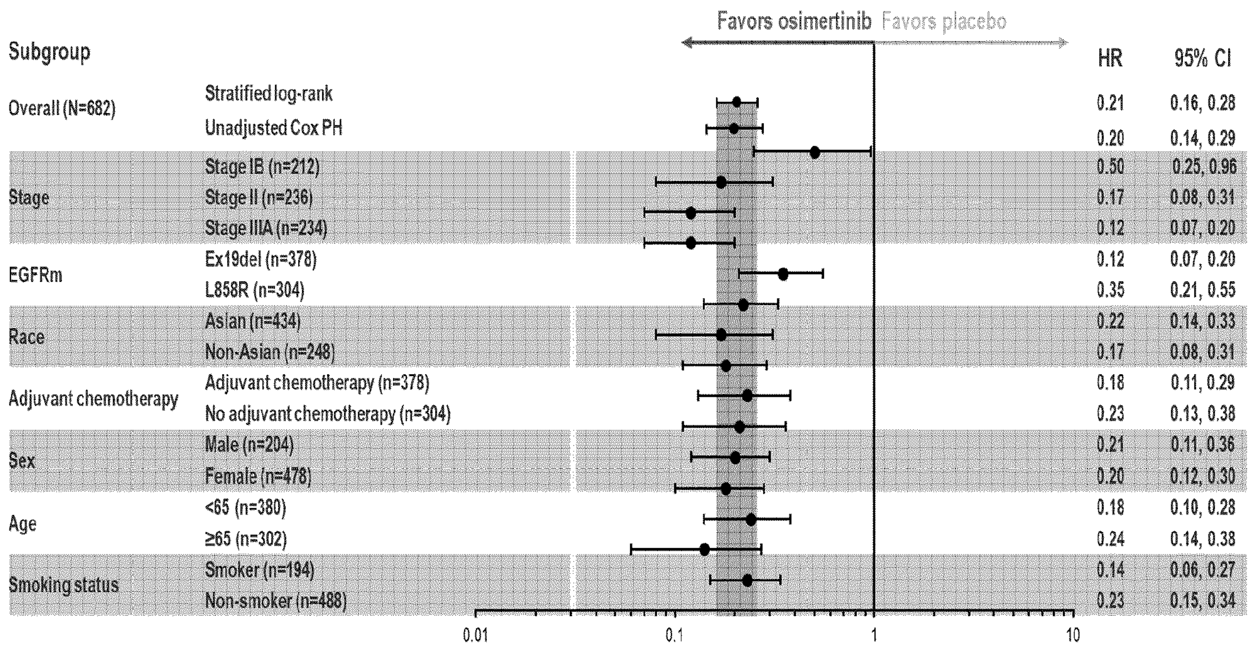


FIGURE 6

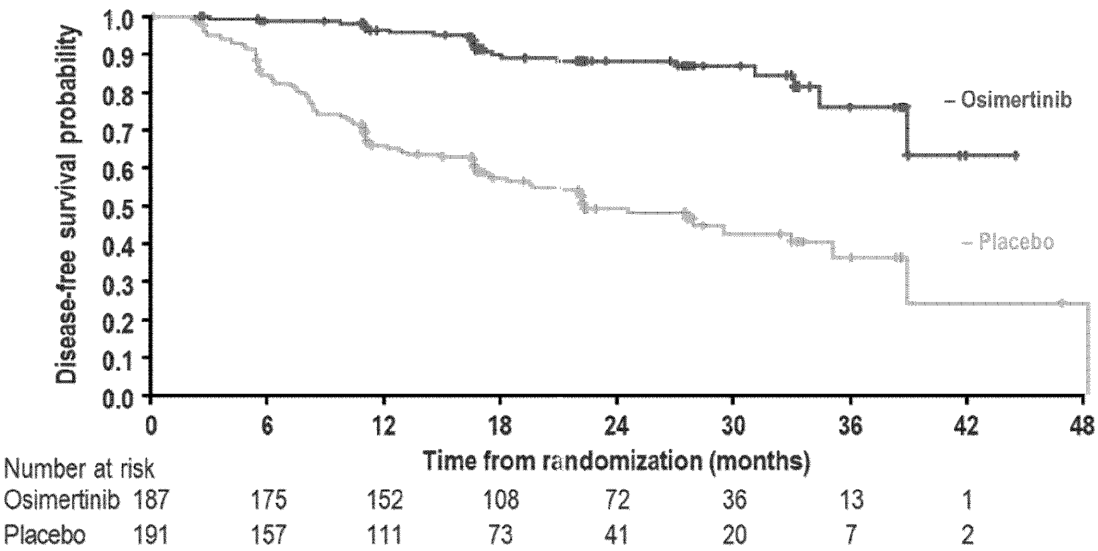


FIGURE 7

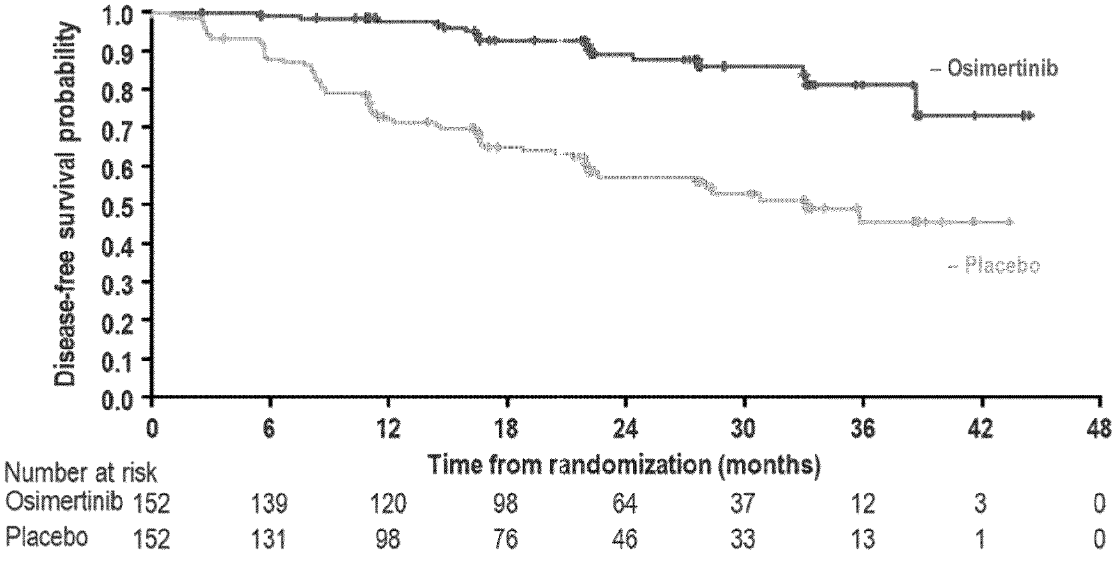


FIGURE 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013014448 A
- WO 2015175632 A
- WO 2016054987 A
- WO 2016015453 A
- WO 2016094821 A
- WO 2016070816 A
- WO 2016173438 A
- WO 2015101791 A
- WO 2014135876 A
- WO 2016060443 A
- US 2014038940 A
- WO 201615453 A
- WO 0250043 A
- WO 2015027222 A
- WO 201694821 A
- WO 2005107758 A
- WO 2013184757 A


### Non-patent literature cited in the description


- **PIGNON et al.** *J Clin Oncol*, 2008, vol. 26, 3552-9
- *Science*, 2004, vol. 304, 1497-1500
- *New England Journal of Medicine*, 2004, vol. 350, 2129-2139
- *J. Thorac. Oncol.*, 2010, 1551-1558
- **CHENG et al.** *Lung Cancer*, 2019, vol. 137, 7-13
- **ZHONG et al.** *Lancet Oncol*, 2018
- **SULLIVAN et al.** *Therapeutic Advances in Respiratory Disease*, 2016, vol. 10 (6), 549-565
- *N. Engl. J Med.*, 2018, vol. 378, 113-25
- *N Engl J Med.*, 2020, vol. 382 (1), 41-50
- *J Clin Oncol.*, 2018, vol. 36 (33), 3290-7
- *Clinical Lung Cancer*, 2018, vol. 19 (4), e533-36
- **BERRY et al.** *Statistics in Medicine*, 1991, vol. 10, 749-55
- **SELKE ; SIEGMUND**. *Biometrika*, 1983, vol. 70, 315-26